# EUROPEAN PATENT SPECIFICATION

(11) **EP 3 996 701 B1**
(45) Date of publication and mention of the grant of the patent: **18.09.2024**
(21) Application number: 20836721.9
(22) Date of filing: 09.07.2020
(51) Int. Cl.: A61K 31/216, A01N 57/12, C07D 273/04

(54) **A PROCESS FOR ISOLATING S-ISOMER CRYSTALS OF INDOXACARB**
VERFAHREN ZUR ISOLIERUNG VON S-ISOMER-KRISTALLEN VON INDOXACARB
PROCÉDÉ D'ISOLEMENT DE CRISTAUX D'ISOMÈRE S D'INDOXACARB

(30) Priority: 10.07.2019 IN 201921027684
(43) Date of publication of application: 18.05.2022
(73) Proprietor: Gharda Chemicals Limited, Ratnagiri, Maharashtra 415722 (IN)
(72) Inventor: GHARDA, Keki Hormusji, Mumbai- Maharashtra 400050 (IN); SHENOY, Diwakar K, Ratnagiri- Maharashtra 415722 (IN); SHET, Laxminarayan S, Ratnagiri- Maharashtra 415722 (IN); SAMANGADKAR, Yatin S, Ratnagiri- Maharashtra 415722 (IN); GOGAVALE, Ashish C, Ratnagiri- Maharashtra 415722 (IN)
(74) Representative: Linage González, Rafael
(86) International application number: PCT/IB2020/056461
(87) International publication number: WO 2021/005549

(56) References cited:
- EP-B1- 2 421 861
- WO-A1-02/06202
- CN-A- 104 311 502
- CN-A- 105 111 164

## Description

### FIELD

The present disclosure relates to a process for isolating S-isomer crystals of Indoxacarb with specific purity.

### DEFINITION

As used in the present disclosure, the following terms are generally intended to have the meaning as set forth below, except to the extent that the context in which they are used indicate otherwise.

**Equilibration** refers to a process of allowing an enantiomeric mass under stirring to attain equilibrium, for obtaining a specific enantiomeric ratio.

**Crude Indoxacarb** refers to Indoxacarb obtained from a manufacturing unit, comprising optically active isomer of Indoxacarb (S-isomer) and optically inactive isomer of Indoxacarb (R-isomer) along with impurities. Crude Indoxacarb is either amorphous or crystalline in nature. The impurities are inert in nature comprising compounds such as oxadiazine precursor, oil and the like. The amount of impurities present in crude Indoxacarb is in the range of 3 wt. % to 15 wt.%.

### BACKGROUND

The background information herein below relates to the present disclosure but is not necessarily prior art.

Indoxacarb is known to have pesticidal activity and is especially effective against lepidopteran larvae. Indoxacarb is an oxadiazine compound exhibiting stereoisomerism, wherein (+)-S-isomer has insecticidal activity whereas the R-isomer is inactive. However, the isolation process to obtain the S-isomer crystals of Indoxacarb with high purity is tedious and expensive.

Therefore, there is felt a need, for an efficient process for isolating S-isomer crystals of Indoxacarb, which mitigates the drawbacks mentioned hereinabove.

### OBJECTS

Some of the objects of the present disclosure, which at least one embodiment herein satisfies, are as follows.

It is an object of the present disclosure to ameliorate one or more problems of the prior art or to at least provide a useful alternative.

Another object of the present disclosure is to isolate S-isomer crystals of Indoxacarb having comparatively high purity.

Still another object of the present disclosure is to provide an efficient process for isolating S-isomer crystals of Indoxacarb having comparatively high purity.

Other objects and advantages of the present disclosure will be more apparent from the following description, which is not intended to limit the scope of the present disclosure.

### SUMMARY

The present disclosure provides a process for isolating S-isomer crystals of Indoxacarb having purity in the range of 98% to 99%. The process comprises mixing crude Indoxacarb with a solvent to obtain a slurry, wherein the crude Indoxacarb comprises impurities in an amount in the range of 3 wt.% to 15 wt.%. The slurry is heated to a temperature in the range of 64 °C to 80 °C to obtain a heated slurry. The heated slurry is cooled to a temperature in the range of 40 °C to 51 °C to allow crystallization of racemic Indoxacarb and obtain a warm slurry comprising crystals of the racemic Indoxacarb. The warm slurry is filtered at a temperature in the range of 40 °C to 51 °C to isolate a mass comprising racemic Indoxacarb crystals and obtain a filtrate. The filtrate is cooled to a temperature in the range of 10 °C to 15 °C to initiate crystallization followed by equilibrating under stirring for a time period in the range of 4 hours to 6 hours to allow complete crystallization to obtain a mixture comprising S-isomer crystals of Indoxacarb. The mixture is filtered to isolate the S-isomer crystals of Indoxacarb. The crystals are washed and dried to obtain S-isomer crystals of Indoxacarb having purity in the range of 98% to 99%.

### BRIEF DESCRIPTION OF THE ACCOMPANYING DRAWING

The present disclosure will now be described with the help of the accompanying drawing, in which:
**Figure 1** illustrates Differential scanning calorimetry (DSC) graph of crude Indoxacarb (R:S ratio - 24:76);
**Figure 2** illustrates Differential scanning calorimetry (DSC) graph of sample 1A (racemic mixture), in accordance with the process of the present disclosure;
**Figure 3** illustrates Differential scanning calorimetry (DSC) graph of sample 2A (S-isomer of Indoxacarb), in accordance with the process of the present disclosure;
**Figure 4** illustrates X-Ray Diffraction (XRD) data of crude Indoxacarb (R:S ratio - 24:76);
**Figure 5** illustrates X-Ray Diffraction (XRD) data of sample 1A (racemic mixture), in accordance with the process of the present disclosure; and
**Figure 6** illustrates X-Ray Diffraction (XRD) data of sample 2A (S-isomer of Indoxacarb), in accordance with the process of the present disclosure.

### DETAILED DESCRIPTION

Embodiments, of the present disclosure, will now be described with reference to the accompanying drawing.

Embodiments are provided so as to thoroughly and fully convey the scope of the present disclosure to the person skilled in the art. Numerous details, are set forth, relating to specific components, and methods, to provide a complete understanding of embodiments of the present disclosure. It will be apparent to the person skilled in the art that the details provided in the embodiments should not be construed to limit the scope of the present disclosure. In some embodiments, well-known processes, well-known apparatus structures, and well-known techniques are not described in detail.

The terminology used, in the present disclosure, is only for the purpose of explaining a particular embodiment and such terminology shall not be considered to limit the scope of the present disclosure. As used in the present disclosure, the forms "a," "an," and "the" may be intended to include the plural forms as well, unless the context clearly suggests otherwise. The terms "comprises," "comprising," "including," and "having," are open ended transitional phrases and therefore specify the presence of stated features, integers, steps, operations, elements, modules, units and/or components, but do not forbid the presence or addition of one or more other features, integers, steps, operations, elements, components, and/or groups thereof. The particular order of steps disclosed in the method and process of the present disclosure is not to be construed as necessarily requiring their performance as described or illustrated. It is also to be understood that additional or alternative steps may be employed.

The terms first, second, third, etc., should not be construed to limit the scope of the present disclosure as the aforementioned terms may be only used to distinguish one element, component, region, layer or section from another component, region, layer or section. Terms such as first, second, third etc., when used herein do not imply a specific sequence or order unless clearly suggested by the present disclosure.

Indoxacarb is a substituted oxadiazine compound having a single chiral center as shown in the structure (I) below. Indoxacarb is an important pesticidal compound showing improved properties in its crystalline form than in the amorphous form.

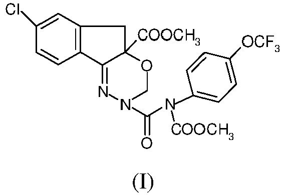

The insecticidal activity of Indoxacarb is mainly attributed to the (+)-S-isomer whereas the R-isomer is inactive. Conventional processes of obtaining S-isomer crystals of Indoxacarb is tedious and expensive.

The present disclosure provides a simple and cost effective process for isolating S-isomer crystals of Indoxacarb having comparatively high purity.

Particularly, the present disclosure provides a process for isolating S-isomer crystals of Indoxacarb having purity in the range of 98% to 99%. The process is described in detail herein below.

Crude Indoxacarb is mixed with a solvent to obtain a slurry. The crude Indoxacarb comprises impurities in an amount in the range of 3 wt.% to 15 wt.%.

The crude Indoxacarb, obtained from a manufacturing unit, comprises optically active isomer of Indoxacarb (S-isomer) and optically inactive isomer of Indoxacarb (R-isomer) along with impurities. The impurities are inert in nature comprising compounds such as oxadiazine precursor, solvent, oil and the like. The amount of impurities in crude Indoxacarb are in the range of 3 wt. % to 15 wt.%. Crude Indoxacarb is either amorphous or crystalline in nature.

Crude Indoxacarb comprises an enantiomeric mixture having S-isomer in an amount in the range of 73% to 80%. In an embodiment, crude Indoxacarb contains S-isomer in an amount of 75% of the enantiomeric mixture.

In an embodiment, crude Indoxacarb is obtained in an amorphous lump form.

In an exemplary embodiment, crude Indoxacarb has total impurities of 3 wt. % to 15 wt.%.

In accordance with the present disclosure, the solvent is selected from the group consisting of aliphatic alcohols with C₁ to C₁₀ carbon atoms and cyclic alcohols.

Typically, the solvent is selected from the group consisting of methanol, ethanol, 2-propanol, 1-butanol and cyclohexanol. In an exemplary embodiment, the solvent is 2-propanol.

The weight-volume ratio of crude Indoxacarb and the solvent in the mixing step is in the range of 1:0.7 to 1:1.5. In an embodiment, the weight-volume ratio of crude Indoxacarb and the solvent is 1:1.

The weight-volume ratio lesser than 1:0.7 would lead to insufficient solubility whereas a ratio greater than 1:1.5 would lead to the use of excess solvent which not only leads to wastage but also affects the recrystallization of S-isomer during equilibration resulting in comparatively lesser recovery.

In an embodiment, the impurities are mainly inert impurities that get dissolved by the solvent at room temperature.

Further, the slurry is heated to a temperature in the range of 64 °C to 80 °C to obtain a heated slurry. Typically, the slurry is heated to a temperature in the range of 65 °C to 75 °C. In an exemplary embodiment, the solvent used is 2-propanol and the slurry is heated to 75 °C.

The heating of the slurry in the temperature range of 64 °C to 80 °C ensures better solubilization of S-isomer of Indoxacarb in the heated slurry. This range of temperature is important because below 64 °C, solubilization will be ineffective and above 80 °C, there might be loss of solvent in the slurry due to evaporation, depending upon the solvent used.

In the next step, the heated slurry is cooled to a temperature in the range of 40 °C to 51 °C to allow crystallization of racemic Indoxacarb and obtain a warm slurry comprising crystals of the racemic Indoxacarb.

The racemic Indoxacarb has lower solubility than the enantiomeric Indoxacarb and hence crystallizes out preferentially in the cooling step. The removal of racemic Indoxacarb ensures that the inactive R-isomer is completely removed thereby leading to isolation of the S-isomer.

Typically, the heated slurry is cooled to a temperature in the range of 40 °C to 51 °C. In an embodiment, the heated slurry is cooled to 43 °C.

In the next step, the warm slurry is filtered at a temperature in the range of 40 °C to 51 °C to isolate a mass comprising the racemic Indoxacarb crystals and obtain a filtrate.

In an exemplary embodiment, the heated slurry is cooled to 48 °C and the filtration is done at 48 °C to isolate a mass comprising the racemic Indoxacarb crystals.

Further, the filtrate is cooled to a temperature in the range of 9 °C to 16 °C to initiate crystallization followed by equilibrating under stirring for a time period in the range of 3 hours to 7 hours at a temperature in the range of 9 °C to 16 °C to allow complete crystallization to obtain a mixture containing S-isomer crystals of Indoxacarb.

The cooling of the filtrate is done by using external source of cooling. The step of cooling the filtrate initiates crystallization of the S-isomer of Indoxacarb. Further, equilibration is the process of allowing a solution to attain equilibrium for obtaining complete crystallization. In accordance with the present disclosure, the equilibration step is very important for allowing complete crystallization of the S-isomer crystals, while the impurities and some amount of S-isomer stay in the filtrate.

Typically, the cooling of the filtrate and the equilibration of the first mixture is done at a temperature in the range of 10 °C to 15 °C.

In an embodiment, the filtrate is cooled to 15 °C and the equilibration of the first mixture is done at 15 °C.

Typically, the equilibration is done for a time period in the range of 4 hours to 6 hours. In an embodiment, the equilibration is done for 6 hours.

In the next step, the mixture is filtered to isolate S-isomer crystals of Indoxacarb. The crystals are washed and dried to obtain the S-isomer crystals of Indoxacarb having purity in the range of 98% to 99%.

The step of washing the crystals is done using a fluid medium selected from the group consisting of methanol, ethanol, 2-propanol, t-butanol, 1-butanol and cyclohexanol. The washing of the crystals with the fluid medium ensures complete removal of the impurities in the filtrate, thereby leading to higher purity. In an embodiment, the crystals are washed using 2-propanol.

The step of drying the mass is done under vacuum at a temperature in the range of 39 °C to 45 °C. The temperature range of drying ensures the removal of the fluid medium from the crystals. In an embodiment, the drying is done at 40 °C.

In one embodiment, the S-isomer crystals of Indoxacarb obtained by the process of the present disclosure has a purity of 98%. In another embodiment, S-isomer crystals of Indoxacarb obtained by the process of the present disclosure has a purity of 99%.

In an exemplary embodiment, crude Indoxacarb is mixed with 2-propanol to obtain a slurry, wherein the crude Indoxacarb comprises impurities in an amount of 14 wt.%. The slurry is heated to 75 °C to obtain a heated slurry. The heated slurry is cooled to 48 °C to allow crystallization of racemic Indoxacarb and obtain a warm slurry comprising crystals of racemic Indoxacarb. The warm slurry is filtered at 48 °C, to isolate a mass comprising the racemic Indoxacarb crystals and obtain a filtrate. The filtrate is cooled to 15 °C to initiate crystallization followed by equilibration under stirring for 6 hours at 15 °C to allow complete crystallization to obtain S-isomer crystals of Indoxacarb. The mixture is filtered to isolate the S-isomer crystals of Indoxacarb. The crystals are washed with 2-propanol and dried at 40 °C under vacuum, to obtain S-isomer crystals of Indoxacarb having purity of 98%.

The commercial sample of Indoxacarb comprises 1:3 ratio of R:S isomers. The conventional process for isolating S-isomer is tedious and expensive. The process of the present disclosure provides a simple and economical process for isolation of the S-isomer crystals of Indoxacarb.

The foregoing description of the embodiments has been provided for purposes of illustration and not intended to limit the scope of the present disclosure. Individual components of a particular embodiment are generally not limited to that particular embodiment, but, are interchangeable. Such variations are not to be regarded as a departure from the present disclosure, and all such modifications are considered to be within the scope of the present disclosure.

The present disclosure is further described in light of the following laboratory scale experiments which are set forth for illustration purpose only and not to be construed for limiting the scope of the disclosure. These laboratory scale experiments can be scaled up to industrial/commercial scale and the results obtained can be extrapolated to industrial/commercial scale.

### Experimental Details

### Experiment 1: Isolation of S-isomer crystals of Indoxacarb (Samples 2A-2F)

### Example a: Isolation of racemic Indoxacarb (Sample 1A) and S-isomer of Indoxacarb (Sample 2A)

Crude Indoxacarb (100 g) having purity of 86% (impurities of 14 wt.%) and R: S isomer ratio= 24:76, was placed in a vessel equipped with stirrer and mixed with Isopropyl alcohol (100 ml) to obtain a slurry. The slurry was heated to 75 °C to obtain a heated slurry, which was then cooled to 48 °C to allow complete crystallization of racemic Indoxacarb and obtain a warm slurry comprising crystals of the racemic Indoxacarb. The warm slurry was then filtered at 48 °C to isolate crystals of racemic Indoxacarb and obtain a filtrate. The racemic Indoxacarb crystals were dried (43 g) and used for further analysis (Sample 1A). The filtrate was cooled to 15 °C to initiate crystallization followed by equilibrating under stirring for 6 hours to allow complete crystallization to obtain a mixture comprising S-isomer crystals of Indoxacarb. The mixture was filtered at 15 °C to isolate the S-isomer crystals of Indoxacarb. The crystals were washed with isopropyl alcohol and then dried under vacuum at 40 °C to get S-isomer crystals of Indoxacarb (Sample 2A) (43g) with purity of 98%.

### Example b: Isolation of racemic Indoxacarb (Sample 1B) and S-isomer of Indoxacarb (Sample 2B)

Crude Indoxacarb (100 g) having purity of 87% (impurities of 13 wt.%) and R: S isomer ratio=25:75, was placed in a vessel equipped with stirrer and mixed with methanol (100 ml) to obtain a slurry. The slurry was heated to 65 °C to obtain a heated slurry, which was then cooled to 43 °C to allow complete crystallization of racemic Indoxacarb and obtain a warm slurry comprising crystals of the racemic Indoxacarb. The warm slurry was then filtered at 43 °C to isolate racemic Indoxacarb and obtain a filtrate. The racemic Indoxacarb crystals were dried (46 g) and used for further analysis (Sample 1B). The filtrate was cooled to 13 °C to initiate crystallization followed by equilibrating under stirring for 6 hours to allow complete crystallization to obtain a mixture comprising S-isomer crystals of Indoxacarb. The mixture was filtered at 13 °C to isolate the S-isomer crystals of Indoxacarb. The crystals were washed with isopropyl alcohol (25 ml) and then dried under vacuum at 40 °C to get S-isomer crystals of Indoxacarb (Sample 2B) (42 g) with purity of 99%.

### Example c: Isolation of racemic Indoxacarb (Sample 1C) and S-isomer of Indoxacarb (Sample 2C)

Crude Indoxacarb (100 g) having purity of 85 % (impurities of 15 wt.%) and R: S isomer ratio=25:75, was placed in a vessel equipped with stirrer and mixed with ethanol (100 ml) to obtain a slurry. The slurry was heated to 70 °C to obtain a heated slurry, which was then cooled to 42 °C to allow complete crystallization of racemic Indoxacarb and obtain a warm slurry comprising crystals of the racemic Indoxacarb. The warm slurry was then filtered at 43 °C to isolate racemic Indoxacarb and obtain a filtrate. The racemic Indoxacarb crystals were dried (44 g) and used for further analysis (Sample 1C). The filtrate was cooled to 13 °C to initiate crystallization followed by equilibrating under stirring for 6 hours to allow complete crystallization to obtain a mixture comprising S-isomer crystals of Indoxacarb. The mixture was filtered at 13 °C to isolate the S-isomer crystals of Indoxacarb. The crystals were washed with cold ethanol (25 ml) and then dried under vacuum at 42 °C to get S-isomer crystals of Indoxacarb (Sample 2C) (42 g) with purity of 98.5 %.

### Example d: Isolation of racemic Indoxacarb (Sample 1D) and S-isomer of Indoxacarb (Sample 2D)

Crude Indoxacarb (100 g) having purity of 86 % (impurities of 14 wt.%) and R: S isomer ratio=24:76, was placed in a vessel equipped with stirrer and mixed with t-butanol (100 ml) to obtain a slurry. The slurry was heated to 75 °C to obtain a heated slurry, which was then cooled to 45 °C to allow complete crystallization of racemic Indoxacarb and obtain a warm slurry comprising crystals of the racemic Indoxacarb. The warm slurry was then filtered at 45 °C to isolate racemic Indoxacarb and obtain a filtrate. The racemic Indoxacarb crystals were dried (45 g) and used for further analysis (Sample 1D). The filtrate was cooled to 15 °C to initiate crystallization followed by equilibrating under stirring for 4 hours to allow complete crystallization to obtain a mixture comprising S-isomer crystals of Indoxacarb. The mixture was filtered at 13 °C to isolate the S-isomer crystals of Indoxacarb. The crystals were washed with cold t-butanol (25 ml) and then dried under vacuum at 45 °C to get S-isomer crystals of Indoxacarb (Sample 2D) (43 g) with purity of 98 %.

### Example e: Isolation of racemic Indoxacarb (Sample 1E) and S-isomer of Indoxacarb (Sample 2E)

Crude Indoxacarb (100 g) having purity of 96.5 % (impurities of 3.5 wt.%) and R: S isomer ratio=27:73, was placed in a vessel equipped with stirrer and mixed with 2-propanol (80 ml) to obtain a slurry. The slurry was heated to 75 °C to obtain a heated slurry, which was then cooled to 45 °C to allow complete crystallization of racemic Indoxacarb and obtain a warm slurry comprising crystals of the racemic Indoxacarb. The warm slurry was then filtered at 45 °C to isolate racemic Indoxacarb and obtain a filtrate. The racemic Indoxacarb crystals were dried (53 g) and used for further analysis (Sample 1E). The filtrate was cooled to 15 °C to initiate crystallization followed by equilibrating under stirring for 4 hours to allow complete crystallization to obtain a mixture comprising S-isomer crystals of Indoxacarb. The mixture was filtered at 15 °C to isolate the S-isomer crystals of Indoxacarb. The crystals were washed with cold 2-propanol (25 ml) and then dried under vacuum at 45 °C to get S-isomer crystals of Indoxacarb (Sample 2E) (42 g) with purity of 99 %.

### Example f: Isolation of racemic Indoxacarb (Sample 1F) and S-isomer of Indoxacarb (Sample 2F)

Crude Indoxacarb (100 g) having purity of 96.5 % (impurities of 3.5 wt.%) and R: S isomer ratio=20:80, was placed in a vessel equipped with stirrer and mixed with 2-propanol (80 ml) to obtain a slurry. The slurry was heated to 75 °C to obtain a heated slurry, which was then cooled to 50 °C to allow complete crystallization of racemic Indoxacarb and obtain a warm slurry comprising crystals of the racemic Indoxacarb. The warm slurry was then filtered at 50 °C to isolate racemic Indoxacarb and obtain a filtrate. The racemic Indoxacarb crystals were dried (45 g) and used for further analysis (Sample 1F). The filtrate was cooled to 15 °C to initiate crystallization followed by equilibrating under stirring for 4 hours to allow complete crystallization to obtain a mixture comprising S-isomer crystals of Indoxacarb. The mixture was filtered at 13 °C to isolate the S-isomer crystals of Indoxacarb. The crystals were washed with cold 2-propanol (25 ml) and then dried under vacuum at 45 °C to get S-isomer crystals of Indoxacarb (Sample 2F) (51 g) with purity of 99 %.

### Experiment 2: Differential scanning calorimetry (DSC) analysis:

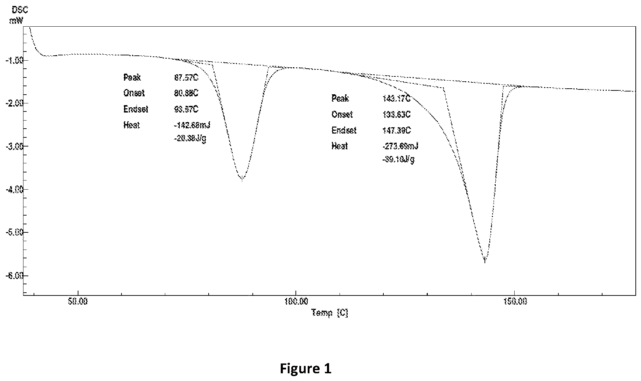

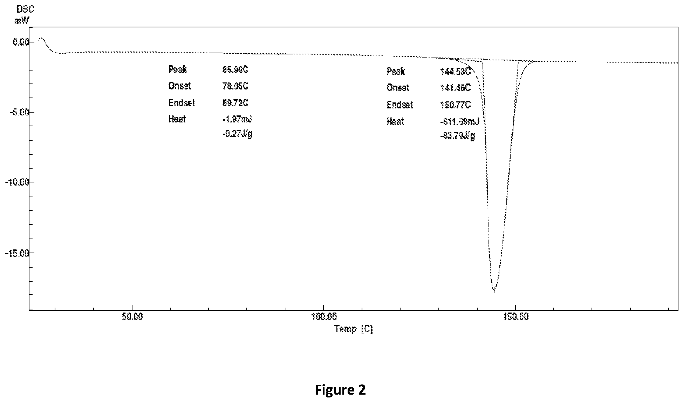

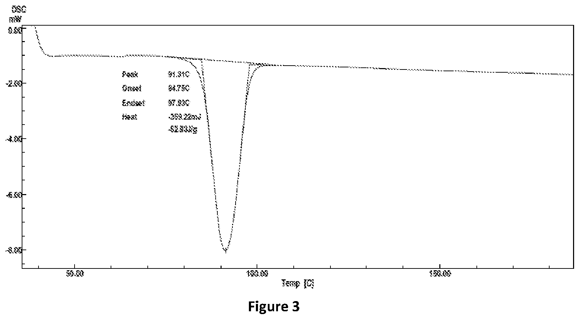

The crude Indoxacarb sample (R:S as 24:76) used in Example a, and the samples 1A (racemic mixture) and 2A (S-isomer of Indoxacarb), as obtained from Example a were subjected to Differential scanning calorimetry (DSC) analysis as shown in Figures 1, 2 and 3 respectively.

The crude Indoxacarb as used in Example a (R:S as 24:76) shows two different peaks in DSC analysis wherein one peak in the range of 88 °C to 93 °C corresponds to melting temperature of S-Indoxacarb and another peak in the range of 143 °C to 147 °C corresponds to melting temperature of racemic Indoxacarb. The sample 1A (racemic Indoxacarb) shows a single peak in DSC corresponding to the melting temperature range of 144.5 °C to 150.7 °C. The sample 2A (S-isomer of Indoxacarb) shows a single peak in DSC corresponding to the melting temperature range of 91.3 °C to 97.9 °C.

### Experiment 3: X-Ray Diffraction (XRD) data:

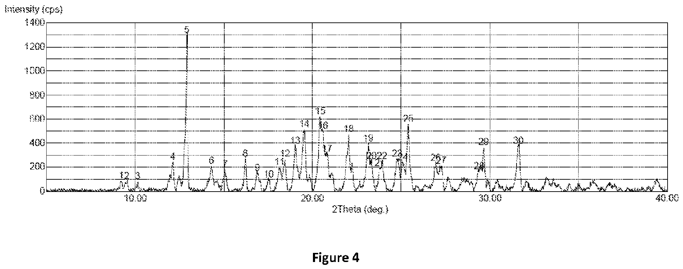

The crude Indoxacarb sample used in Example a (R:S as 24:76), and samples 1A (racemic mixture), 2A (S-isomer of Indoxacarb), as obtained from Example a was subjected to X-Ray Diffraction (XRD) analysis to obtain data as shown in Figures 4, 5 and 6 respectively.

The significant peaks in XRD for the crude Indoxacarb sample used in Experiment 1 (R:S as 24:76), sample 1A and sample 2A, as obtained from Experiment 1 are as summarized in Table 1, Table 2 and Table 3 respectively.

**Table 1: XRD values for crude Indoxacarb**

| Sr. No. | Peak No in Figure 4 | 2θ | Flex Width | d-value | Intensity | I/Io |
|---|---|---|---|---|---|---|
| 1 | 5 | 12.920 | 0.141 | 6.8464 | 1292 | 100 |
| 2 | 12 | 18.450 | 0.200 | 4.8049 | 265 | 21 |
| 3 | 13 | 19.030 | 0.165 | 4.6597 | 375 | 30 |
| 4 | 14 | 19.540 | 0.224 | 4.5392 | 514 | 40 |
| 5 | 15 | 20.430 | 0.153 | 4.3435 | 618 | 48 |
| 6 | 16 | 20.590 | 0.118 | 4.3101 | 498 | 39 |
| 7 | 18 | 22.050 | 0.141 | 4.0279 | 473 | 37 |
| 8 | 19 | 23.150 | 0.224 | 3.8389 | 391 | 31 |
| 9 | 25 | 25.410 | 0.165 | 3.5024 | 554 | 43 |
| 10 | 30 | 31.610 | 0.235 | 2.8281 | 376 | 30 |

**Table 2: XRD values for racemic Indoxacarb (Sample 1A)**

| Sr. No. | Peak No in Figure 5 | 2θ | Flex Width | d-value | Intensity | I/Io |
|---|---|---|---|---|---|---|
| 1 | 2 | 12.100 | 0.141 | 7.3084 | 416 | 31 |
| 2 | 4 | 12.920 | 0.153 | 6.8464 | 1379 | 100 |
| 3 | 13 | 19.570 | 0.165 | 4.5324 | 401 | 30 |
| 4 | 14 | 20.400 | 0.129 | 4.3498 | 955 | 70 |
| 5 | 16 | 22.020 | 0.153 | 4.0333 | 998 | 73 |
| 6 | 17 | 22.250 | 0.106 | 3.9921 | 589 | 43 |
| 7 | 30 | 31.610 | 0.235 | 2.8281 | 376 | 30 |

**Table 3: XRD values for S-isomer of Indoxacarb (Sample 2A)**

| Sr. No. | Peak No in Figure 6 | 2θ | Flex Width | d-value | Intensity | I/Io |
|---|---|---|---|---|---|---|
| 1 | 1 | 9.280 | 0.165 | 9.5220 | 425 | 35 |
| 2 | 5 | 18.180 | 0.153 | 4.8756 | 390 | 32 |
| 3 | 6 | 18.510 | 0.188 | 4.7895 | 1241 | 100 |
| 4 | 7 | 19.090 | 0.200 | 5.6452 | 861 | 70 |
| 5 | 8 | 19.580 | 0.212 | 4.5301 | 606 | 49 |
| 6 | 10 | 20.640 | 0.212 | 4.2997 | 680 | 55 |
| 7 | 11 | 20.870 | 0.153 | 4.2529 | 631 | 51 |
| 8 | 17 | 25.610 | 0.224 | 3.4755 | 412 | 34 |
| 9 | 18 | 27.320 | 0.212 | 3.2617 | 958 | 78 |

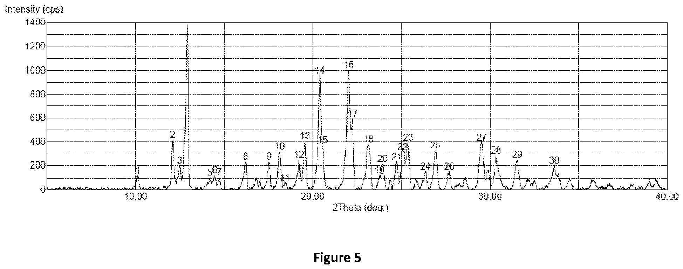

As observed in Figures 4, 5 and Tables 1, 2, the significant peaks in Figure 4 having 2Θ values of 12.920, 19.540, 20.430 and 22.050 are also observed in Figure 5, confirming the isolation of racemic Indoxacarb (sample 1A).

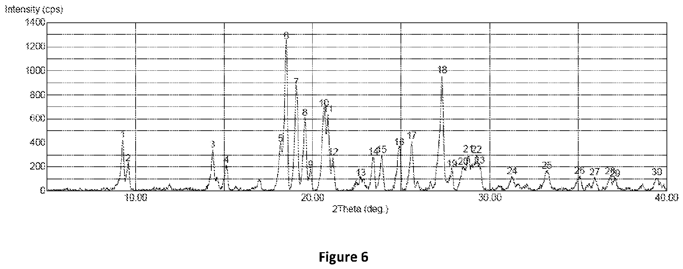

As observed in Figures 4, 6 and Tables 1, 3, the significant peaks in Figure 4 having 2Θ values of 18.450, 19.030, 19.540, 25.410 are also observed in Figure 6, confirming the presence of S-isomer of Indoxacarb in the sample 2A.

The process of the present disclosure is simple and cost effective, wherein S-isomer crystals of Indoxacarb are isolated in comparatively high purity.

### TECHNICAL ADVANCEMENTS

The present disclosure described herein above has several technical advantages including but not limited to the realization of a process for isolating S-isomer crystals of Indoxacarb that:
- provides comparatively high purity; and
- is simple and economical.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The use of the expression "at least" or "at least one" suggests the use of one or more elements or ingredients or quantities, as the use may be in the embodiment of the disclosure to achieve one or more of the desired objects or results.

## Claims

1. A process for isolating S-isomer crystals of Indoxacarb having purity in the range of 98% to 99%, said process comprising the following steps:
a) mixing crude Indoxacarb with a solvent to obtain a slurry, wherein the crude Indoxacarb comprises impurities in an amount in the range of 3 wt.% to 15 wt.%;
b) heating said slurry to a temperature in the range of 64 °C to 80 °C to obtain a heated slurry;
c) cooling said heated slurry to a temperature in the range of 40 °C to 51 °C to allow complete crystallization of racemic Indoxacarb and obtain a warm slurry comprising crystals of the racemic Indoxacarb;
d) filtering said warm slurry at a temperature in the range of 40 °C to 51 °C to isolate a mass comprising racemic Indoxacarb crystals and obtain a filtrate;
e) cooling said filtrate to a temperature in the range of 9 °C to 16 °C to initiate crystallization followed by equilibrating under stirring for a time period in the range of 3 hours to 7 hours to allow complete crystallization to obtain a mixture comprising S-isomer crystals of Indoxacarb;
f) filtering said mixture to isolate said S-isomer crystals of Indoxacarb; and
g) washing and drying said crystals to obtain S-isomer crystals of Indoxacarb having purity in the range of 98% to 99%.

2. The process as claimed in claim 1, wherein the crude Indoxacarb comprises an enantiomeric mixture having S-isomer in an amount in the range of 73% to 80%.

3. The process as claimed in claim 1, wherein the weight-volume ratio of the crude Indoxacarb and the solvent in step (a) is in the range of 1:0.7 to 1:1.5.

4. The process as claimed in claim 1, wherein said solvent in step (a) is selected from the group consisting of aliphatic alcohols with C₁ to C₁₀ carbon atoms and cyclic alcohols.

5. The process as claimed in claim 1 or claim 4, wherein said solvent in step (a) is selected from the group consisting of methanol, ethanol, 2-propanol, t-butanol, 1-butanol and cyclohexanol.

6. The process as claimed in claim 1, wherein said washing in step (g) is done by using a fluid medium selected from the group consisting of methanol, ethanol, 2-propanol, t-butanol, 1-butanol and cyclohexanol.

7. The process as claimed in claim 1, wherein said drying in step (g) is done under vacuum at a temperature in the range of 39 °C to 45 °C.

8. The process as claimed in claim 1, wherein said process comprises the following steps:
a) mixing crude Indoxacarb with a solvent to obtain a slurry, wherein the crude Indoxacarb comprises impurities in an amount of 14 wt.%, wherein the crude Indoxacarb comprises an enantiomeric mixture having S-isomer in an amount of 76%;
b) heating said slurry to 75 °C to obtain a heated slurry;
c) cooling said heated slurry to 48 °C to allow crystallization of racemic Indoxacarb and obtain a warm slurry comprising crystals of racemic Indoxacarb;
d) filtering said warm slurry at 48 °C to isolate a mass comprising racemic Indoxacarb crystals and obtain a filtrate;
e) cooling said filtrate to 15 °C to initiate crystallization followed by equilibrating under stirring for 6 hours at 15 °C to allow complete crystallization to obtain a mixture comprising S-isomer crystals of Indoxacarb;
f) filtering said mixture to isolate said S-isomer crystals of Indoxacarb; and
g) washing and drying said crystals to obtain S-isomer crystals of Indoxacarb having purity of 98 %.

## Patentansprüche

1. Verfahren zum Isolieren von S-Isomerkristallen von Indoxacarb mit einem Reinheitsgrad im Bereich von 98 % bis 99 %, wobei das Verfahren die folgenden Schritte umfasst:
a) Vermischen von Roh-Indoxacarb mit einem Lösungsmittel, um eine Aufschlämmung zu erhalten, wobei das Roh-Indoxacarb Verunreinigungen in einer Menge im Bereich von 3 Gew.-% bis 15 Gew.-% enthält.
b) Aufheizen der Aufschlämmung auf eine Temperatur im Bereich von 64 °C bis 80 °C, um eine aufgeheizte Aufschlämmung zu erhalten;
c) Abkühlen der aufgewärmten Aufschlämmung auf eine Temperatur im Bereich von 40 °C bis 51 °C, um eine vollständige Kristallisierung von racemischem Indoxacarb zu ermöglichen und eine warme Aufschlämmung zu erhalten, die Kristalle von racemischem Indoxacarb enthält;
d) Filtern der warmen Aufschlämmung bei einer Temperatur im Bereich von 40 °C bis 51 °C, um eine Masse zu isolieren, die racemische Indoxacarb-Kristalle enthält, und ein Filtrat zu erhalten;
e) Abkühlen des Filtrats auf eine Temperatur im Bereich von 9 °C bis 16 °C, um unter Rühren die Kristallisierung gefolgt vom ins Gleichgewicht bringen auszulösen, und zwar für einen Zeitraum im Bereich von 3 Stunden bis 7 Stunden, um eine vollständige Kristallisierung zu ermöglichen und ein Gemisch zu erhalten, das S-Isomerkristalle von Indoxacarb enthält;
f) Filtern des Gemischs, um S-Isomerkristalle des Indoxacarb zu isolieren; und
g) Waschen und Trocknen der Kristalle, um S-Isomerkristalle von Indoxacarb mit einem Reinheitsgrad im Bereich von 98 % bis 99 % zu erhalten.

2. Verfahren nach Anspruch 1, wobei das Roh-Indoxacarb ein enantiomeres Gemisch mit S-Isomeren in einer Menge im Bereich von 73 % bis 80 % enthält.

3. Verfahren nach Anspruch 1, wobei das Gewichts-Volumen-Verhältnis des Roh-Indoxacarb und des Lösungsmittels in Schritt (a) im Bereich von 1:0,7 bis 1:1,5 liegt.

4. Verfahren nach Anspruch 1, wobei das Lösungsmittel in Schritt (a) aus einer Gruppe bestehend aus aliphatischen Alkoholen mit C₁- C₁₀ Kohlenstoffatomen und zyklischen Alkoholen gewählt wird.

5. Verfahren nach Anspruch 1 oder Anspruch 4, wobei das Lösungsmittel in Schritt (a) aus der Gruppe bestehend aus Methanol, Ethanol, 2-Propanol, t-Butanol, 1-Butanol und Cyclohexanol gewählt wird.

6. Verfahren nach Anspruch 1, wobei das Waschen in Schritt (g) mit Hilfe eines flüssigen Mediums geschieht, das aus der Gruppe bestehend aus Methanol, Ethanol, 2-Propanol, t-Butanol, 1-Butanol und Cyclohexanol gewählt wird.

7. Verfahren nach Anspruch 1, wobei das Trocknen in Schritt (g) unter Vakuum bei einer Temperatur im Bereich von 39 °C bis 45 °C geschieht.

8. Verfahren nach Anspruch 1, wobei das Verfahren die folgenden Schritte umfasst:
a) Vermischen von Roh-Indoxacarb mit einem Lösungsmittel, um eine Aufschlämmung zu erhalten, wobei das Roh-Indoxacarb Verunreinigungen in einer Menge im Bereich von 14 Gew.-% enthält und wobei das Roh-Indoxacarb ein enantiomeres Gemisch mit S-Isomeren in einer Menge von 76 % enthält;
b) Aufheizen der Aufschlämmung auf 75 °C, um eine aufgeheizte Aufschlämmung zu erhalten;
c) Abkühlen der aufgewärmten Aufschlämmung auf 48 °C, um eine Kristallisierung von racemischem Indoxacarb zu ermöglichen und eine warme Aufschlämmung zu erhalten, die Kristalle von racemischem Indoxacarb enthält;
d) Filtern der warmen Aufschlämmung bei 48 °C, um eine Masse zu isolieren, die racemische Indoxacarb-Kristalle enthält, und ein Filtrat zu erhalten;
e) Abkühlen des Filtrats auf 15 °C, um unter Rühren die Kristallisierung gefolgt vom ins Gleichgewicht bringen auszulösen, und zwar für 6 Stunden bei 15 °C, um eine vollständige Kristallisierung zu ermöglichen und ein Gemisch zu erhalten, das S-Isomerkristalle von Indoxacarb enthält;
f) Filtern des Gemischs, um S-Isomerkristalle des Indoxacarb zu isolieren; und
g) Waschen und Trocknen der Kristalle, um S-Isomerkristalle von Indoxacarb mit einem Reinheitsgrad von 98 % zu erhalten.

## Revendications

1. Procédé d'isolement de cristaux de l'isomère S de l'indoxacarbe d'une pureté comprise entre 98 % et 99 %, comprenant les étapes suivantes :
a) le mélange de l'indoxacarbe brut avec un solvant pour obtenir une boue, dans laquelle l'indoxacarbe brut comprend des impuretés dans une proportion comprise entre 3 % en poids et 15 % en poids ;
b) le chauffage de ladite boue à une température comprise entre 64 °C et 80 °C afin d'obtenir une boue chauffée ;
c) le refroidissement de ladite boue chauffée à une température comprise entre 40 °C et 51 °C pour permettre la cristallisation complète de l'indoxacarbe racémique afin d'obtenir une boue tiède comprenant des cristaux d'indoxacarbe racémique ;
d) le filtrage de ladite boue chaude à une température comprise entre 40 °C et 51 °C afin d'isoler une masse comprenant des cristaux d'indoxacarbe racémique et d'obtenir un filtrat ;
e) le refroidissement dudit filtrat à une température comprise entre 9 °C et 16 °C pour amorcer la cristallisation, suivi d'un équilibrage sous agitation pendant une durée comprise entre 3 heures et 7 heures pour permettre une cristallisation complète afin d'obtenir un mélange comprenant des cristaux de l'isomère S de l'indoxacarbe ;
f) le filtrage dudit mélange pour isoler lesdits cristaux de l'isomère S de l'indoxacarbe ; et
g) le lavage et le séchage desdits cristaux pour obtenir des cristaux de l'isomère S de l'indoxacarbe d'une pureté comprise entre 98 % et 99 %.

2. Procédé selon la revendication 1, dans lequel l'indoxacarbe brut comprend un mélange énantiomérique avec l'isomère S dans une proportion comprise entre 73 % et 80 %.

3. Procédé selon la revendication 1, dans lequel le rapport poids-volume de l'indoxacarbe brut et du solvant à l'étape (a) est compris entre 1:0,7 et 1:1,5.

4. Procédé selon la revendication 1, dans lequel le solvant de l'étape (a) est choisi dans le groupe constitué des alcools aliphatiques à atomes de carbone Ci to C₁₀ et des alcools cycliques.

5. Procédé selon la revendication 1 ou la revendication 4, dans lequel le solvant de l'étape (a) est choisi dans le groupe constitué du méthanol, de l'éthanol, du 2-propanol, du t-butanol, du 1-butanol et du cyclohexanol.

6. Procédé selon la revendication 1, dans lequel le lavage à l'étape (g) est effectué à l'aide d'un milieu liquide choisi dans le groupe constitué du méthanol, de l'éthanol, du 2-propanol, du t-butanol, du 1-butanol et du cyclohexanol.

7. Procédé selon la revendication 1, dans lequel le séchage à l'étape (g) est effectué sous vide à une température comprise entre 39 °C et 45 °C.

8. Procédé selon la revendication 1, dans lequel ledit procédé comprend les étapes suivantes :
a) le mélange de l'indoxacarbe brut avec un solvant pour obtenir une boue, dans laquelle l'indoxacarbe brut comprend des impuretés dans une proportion de 14 % en poids, dans laquelle l'indoxacarbe brut comprend un mélange énantiomérique avec l'isomère S dans une proportion de 76 % ;
b) le chauffage de ladite boue à 75 °C pour obtenir une boue chauffée ;
c) le refroidissement de ladite boue chauffée à 48 °C pour permettre la cristallisation de l'indoxacarbe racémique afin d'obtenir une boue tiède comprenant des cristaux d'indoxacarbe racémique ;
d) le filtrage de ladite boue chaude à une et 48 °C afin d'isoler une masse comprenant des cristaux d'indoxacarbe racémique et d'obtenir un filtrat ;
e) le refroidissement dudit filtrat à 15 °C pour amorcer la cristallisation, suivi d'un équilibrage sous agitation pendant 6 heures à 15 °C pour permettre une cristallisation complète afin d'obtenir un mélange comprenant des cristaux de l'isomère S de l'indoxacarbe ;
f) le filtrage dudit mélange pour isoler lesdits cristaux de l'isomère S de l'indoxacarbe ; et
g) le lavage et le séchage desdits cristaux pour obtenir des cristaux de l'isomère S de l'indoxacarbe d'une pureté de 98 %.
